# EUROPEAN PATENT SPECIFICATION

(11) **EP 2 397 695 B1**
(45) Date of publication and mention of the grant of the patent: **11.02.2015**
(21) Application number: 09840048.4
(22) Date of filing: 16.12.2009
(51) Int. Cl.: F04C 5/00, F04B 43/00, F04B 43/12

(54) **TUBE PUMP**
SCHLAUCHPUMPE
POMPE À TUBE

(30) Priority: 16.02.2009 JP 2009033071
(43) Date of publication of application: 21.12.2011
(73) Proprietor: NIPRO CORPORATION, Kita-ku Osaka-shi, Osaka 531-8510 (JP); Shibuya Kogyo Co., Ltd., Ishikawa 920-8681 (JP)
(72) Inventor: SONODA Yoshiyuki, Osaka-shi Osaka 531-8510 (JP); YANAGIMOTO Yoji, Osaka-shi Osaka 531-8510 (JP); YAMAGUCHI Akinobu, Osaka-shi Osaka 531-8510 (JP); SAWADA Toshiharu, Kanazawa-shi Ishikawa 920-8681 (JP); ITOH Yoichi, Kanazawa-shi Ishikawa 920-8681 (JP)
(74) Representative: Oxley, Rachel Louise
(86) International application number: PCT/JP2009/070973
(87) International publication number: WO 2010/092729

(56) References cited:
- EP-A1- 0 672 830
- JP-A- 11 008 922
- JP-A- 2000 345 971
- JP-A- 2001 295 773
- JP-A- 2005 061 245
- JP-A- 2006 046 508
- JP-A- 2007 198 150
- JP-B2- 2 593 058
- US-A1- 2008 085 200
- US-B1- 6 406 267

## Description

### Technical Field

The present invention relates to a tube pump, and more specifically relates to an improvement of a tube pump that pumps a fluid, such as blood or saline, for example.

### Background Art

Conventionally, tube pumps configured to pump a fluid by squeezing a tube by means of a rotor that is being rotated have publicly been known, and in particular, tube pumps are used as blood pumps or fluid replacement pumps in dialysis apparatuses used for dialysis treatment.

A dialysis apparatus using such a tube pump requires work for attaching/detaching tubes to/from the tube pump or a dialyzer before the start and after the end of a dialysis treatment. In a blood circuit including a plurality of tubes, the order and direction of the tubes attached to the tube pump are determined, and thus, complicated work is necessary for attaching/detaching the blood circuit to/from the tube pump.

Therefore, in order to simplify such complicated work, it has been proposed that tubes be attached to a housing of a pump via connection means such as a connector (for example, Patent Literatures 1 and 2).

Furthermore, there are publicly known tube pumps in which a holder is secured to a pair of supply and discharge tubes connected to opposite ends of a curved tube and the holder is attached to a housing (for example, Patent Literatures 3 and 4).

### Prior Art Documents

### Patent Literature

Patent Literature 1: Japanese Patent Laid-Open No. 60-75785
Patent Literature 2: National Publication of International Patent Application No. 2007-537390
Patent Literature 3: Japanese Patent Laid-Open No. 2007-198150
Patent Literature 4: Japanese Patent No. 2593058

US 6406267B discloses a peristaltic pump having a feed tube and a return tube, each tube having a connector with a respective flange which is received in a respective slot on a yoke for preventing axial movement of the tubes, within the yoke.
EP 0672830A discloses a tube fixation device for a roller pump which is separable from the pump head of the roller pump. The fixation device has two grooves for receiving clamping elements which each have a bore for receiving the pump tubing.
US 2008/0085200-A1 discloses a pump with a housing and a tubing holder and connecting element for fixing the pump tubing in the housing. The pump tubing is fixed in receptacles in the holder and the connecting element forms a snap-fit connection with the holder to seal the tubing within the housing.
JP 2005-061245 discloses a tube pump for a printer. The tube of the tube pump comprises an engagement projection which is received in a recess on the housing of the pump.

### Summary of Invention

### Problems to be Solved by the Invention

However, each of the connectors described in Patent Literatures 1 and 2 above is fabricated by subjecting a synthetic resin to one-piece molding, and a blood passage, which is a part of a blood circuit, is provided within the connector. Therefore, the tube pumps described in Patent Literatures 1 and 2 suffer the problem of making the structure of a mold for fabricating the connector complicated, which in turn increases the manufacturing costs of the tube pumps.

Meanwhile, in Patent Literatures 3 and 4, a holder is secured to tubes in advance in such a manner that the holder is perpendicular to the tubes, requiring complicated work for such securing. In addition, it is necessary to carefully handle the plurality of tubes after attachment of the holder thereto so as not to be entangled with a holder for other tubes, resulting in suffering the drawback of making handling and maintenance of the tubes complicated.

### Means for Solving the Problems

In view of such circumstances, the present invention provides a tube pump according to claim 1.

### Advantageous Effects of Invention

The invention according to claim 1 enables the work for attaching/detaching the duct to/from the housing to be easily performed. Also, since no fluid passage is provided in the holder itself, the configuration of the holder can be simplified compared to the conventional art. Thus, the configuration of a mold for fabricating a holder can be simplified compared to the conventional art, which in turn enables a decrease in manufacturing costs of the tube pump compared to the conventional art.

### Brief Description of Drawings

[Figure 1] Figure 1 is a front view of a tube pump according to an embodiment of the present invention.
[Figure 2] Figure 2 is a perspective view of the tube pump illustrated in Figure 1.
[Figure 3] Figure 3 is a perspective view of the tube pump in Figure 2 with a cut major portion.
[Figure 4] Figure 4 is a perspective view of the duct and the holder illustrated in Figure 1 before engagement.
[Figure 5] Figure 5 is a perspective view of a state in which the duct and the holder in Figure 4 are engaged with each other.
[Figure 6] Figure 6 is a perspective view of the duct and the holder illustrated in Figure 5 viewed from the lower surface side.
[Figure 7] Figure 7 provides diagrams of a process for attaching a looped large-diameter tube to a cylindrical sidewall of a housing in the tube pump in Figure 1,
Figure 7(a) illustrates a state immediately before fitting the large-diameter tube onto the cylindrical sidewall, Figure 7(b) illustrates a state in which the large-diameter tube is being fitted onto the cylindrical sidewall, and Figure 7(c) illustrates a state in which the large-diameter tube has been fitted on the cylindrical sidewall.
[Figure 8] Figure 8 provides diagrams of a process for detaching a large-diameter tube from a cylindrical sidewall of a housing in the tube pump in Figure 1,
Figure 8(a) illustrates a state immediately before detaching the large-diameter tube from the cylindrical sidewall, Figure 8(b) illustrates a state in which the large-diameter tube is being detached from the cylindrical sidewall, and Figure 8(c) illustrates a state in which the large-diameter tube has been detached from the cylindrical sidewall.
[Figure 9] Figure 9 is a configuration diagram of a major portion of a tube pump according to another embodiment of the present invention.

### Mode for Carrying Out the Invention

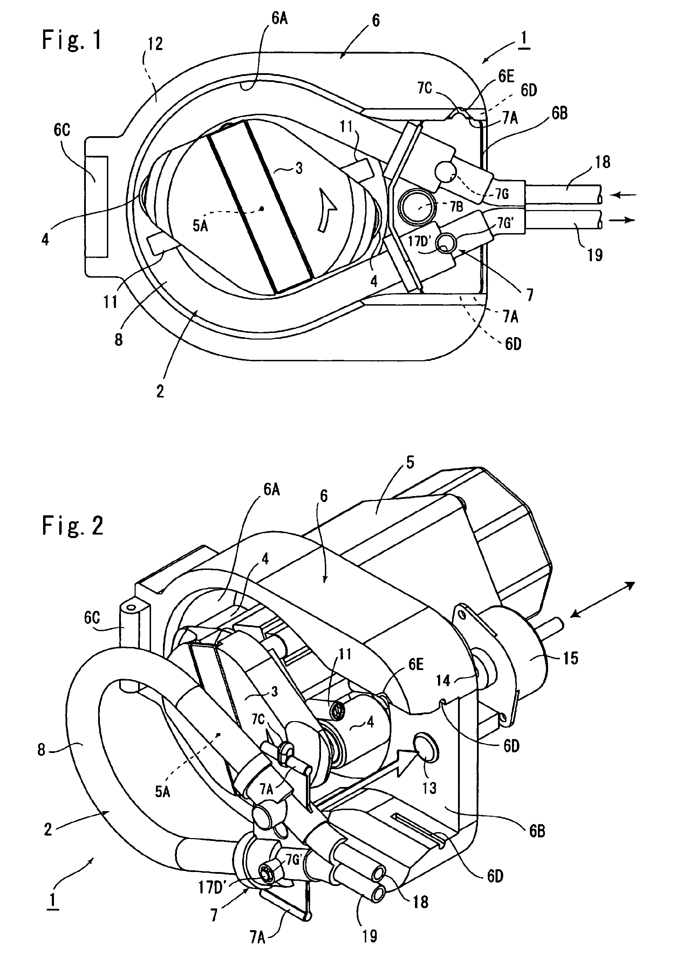

Hereinafter, the present invention will be described based on the illustrated embodiments. In Figures 1 to 3, reference numeral 1 denotes a tube pump, and the tube pump 1 is configured to pump a fluid (blood) by squeezing a duct 2, which made of a soft tube, by means of rollers 4 and 4 in a rotor 3. The tube pump 1 is used as a blood pump for a dialysis apparatus, and is provided on a front surface of a main body portion of the dialysis apparatus. Figure 1 is a front view of the tube pump 1 provided on the front surface of the dialysis apparatus, and Figure 2 is a perspective view of the tube pump 1.

The tube pump 1 according to the present embodiment includes: a motor 5 arranged in the main body portion of the dialysis apparatus, the motor 5 being supported in such a manner that a drive shaft 5A extends horizontally; a rotor 3 with a center portion thereof coupled to a tip of the drive shaft 5A of the motor 5, the rotor 3 being rotated on a vertical surface with the drive shaft 5A as a rotational center thereof; a housing 6 vertically arranged in the main body portion of the dialysis apparatus, the housing 6 surrounding the rotor 3; a duct 2 including a plurality of tubes connected in series, the duct having flexibility: and a holder 7 that is attachable/detachable to/from the housing 6 in a state in which the holder 7 holds the duct 2.

In the rotor 3, the paired rollers 4 and 4 are rotatably supported with reference to respective axes thereof at positions shifted from each other by 180 degrees in a rotational direction thereof, guide pins 11 and 11, which extend radially, are arranged anterior to the respective rollers 4 in the rotational direction of the rollers 4. Each of the guide pins 11 includes a rod-shaped member arranged in a direction perpendicular to the drive shaft 5A and a cylindrical member rotatably attached to an outer peripheral portion of the rod-shaped member. The guide pins 11 are arranged at positions exterior to the positions of the respective rollers 4 on the front surface of the main body portion of the dialysis apparatus (see Figure 2). Accordingly, upon the drive shaft 5A of the motor 5 being rotated by a non-illustrated control device, the rotor 3 is rotated counterclockwise with the drive shaft 5A as the rotational center thereof in Figure 1, and in such case, the guide pins 11 are moved a little ahead of the respective rollers 4.

Although described in details later, in the present embodiment, when the respective guide pins 11 are rotated counterclockwise together with the rotor 3, the respective guide pins 11 are rotated counterclockwise in a state in which the guide pins 11 are in contact with an outer surface of a large-diameter tube 8, which is a curved portion of the duct 2, on the outer surface side of the dialysis apparatus, enabling automatic attachment of the large-diameter tube 8 of the duct 2 between a cylindrical sidewall 6A of the housing 6 and the rollers 4 and 4. Also, the rotor 3 is rotated in a state in which the guide pins 11 are made to burrow into between the large-diameter tube 8 attached to the cylindrical sidewall 6A as described above and a cylindrical bottom surface of the housing 6, enabling automatic detachment of the large-diameter tube 8 of the duct 2 from the cylindrical sidewall 6A of the housing 6. In such automatic attachment/detachment of the duct 2 to/from the cylindrical sidewall 6A, the cylindrical member of each guide pin 11 rotates (spins) as a result of coming into contact with the outer surface of the large-diameter tube 8, enabling smooth attachment/detachment of the large-diameter tube 8 between the cylindrical sidewall 6A and the rollers 4 and 4.

Although in the present embodiment, a pair of rollers 4 and 4 and a pair of guide pins 11 and 11 are provided in the rotor 3, at least one roller 4 and at least one guide pin 11 may be provided in one rotor 3.

The housing 6 according to the present embodiment includes the cylindrical sidewall 6A formed in a cylindrical shape surrounding the rotor 3, and a notch portion 6B, which is continuous from the cylindrical sidewall 6A and extends toward one side in a horizontal direction. Also, the housing 6 is provided with a transparent access cover 12 made of a synthetic resin, in order to cover the cylindrical sidewall 6A and the notch portion 6B on the outside of the front surface of the dialysis apparatus. The access cover 12 is opened/closed via a hinge portion 6C located on the left side of the front surface of the tube pump 1.

When an operator opens the access cover 12, front surface sides of the cylindrical sidewall 6A and the notch portion 6B are exposed to the outside. Then, in such a state, the operator attaches the holder 7 holding the duct 2 to the notch portion 6B of the housing 6, and then, as described above, makes the guide pins 11 and 11 rotate by means of the rotor 3, thereby automatically attaching the large-diameter tube 8 of the duct 2 between the cylindrical sidewall 6A and the rollers 4 and 4.

At upper and lower sidewalls of the notch portion 6B of the housing 6, engagement grooves 6D and 6D extending in a longitudinal direction (horizontal direction) are formed, respectively, and paired engagement portions 7A and 7A formed on the holder 7 side are engaged with the respective engagement grooves 6D and 6D.

Furthermore, at a predetermined position in the upper sidewall of the notch portion 6B, a positioning recess portion 6E is formed in a direction perpendicular to the engagement grooves 6D, and meanwhile, at a predetermined position in the longitudinal direction of the engagement portion 7A on the upper side of the holder 7, a positioning projection 7C is formed.

In the present embodiment, the operator fits the holder 7 onto the duct 2 to hold the duct 2, and when the operator inserts the positioning projection 7C of the holder 7 in such a state into the positioning recess portion 6E of the housing 6 and then fits and pushes the holder 7 into the notch portion 6B, thereby the engagement portions 7A and 7A of the holder 7 being engaged with the engagement grooves 6D and 6D. Consequently, the holder 7 is attached to the notch portion 6B of the housing 6. In a state in which the attachment of the holder 7 has been completed, the holder 7 and parts of the duct 2 held by the holder 7 are positioned relative to the housing 6 in vertical, horizontal and inside-outside directions.

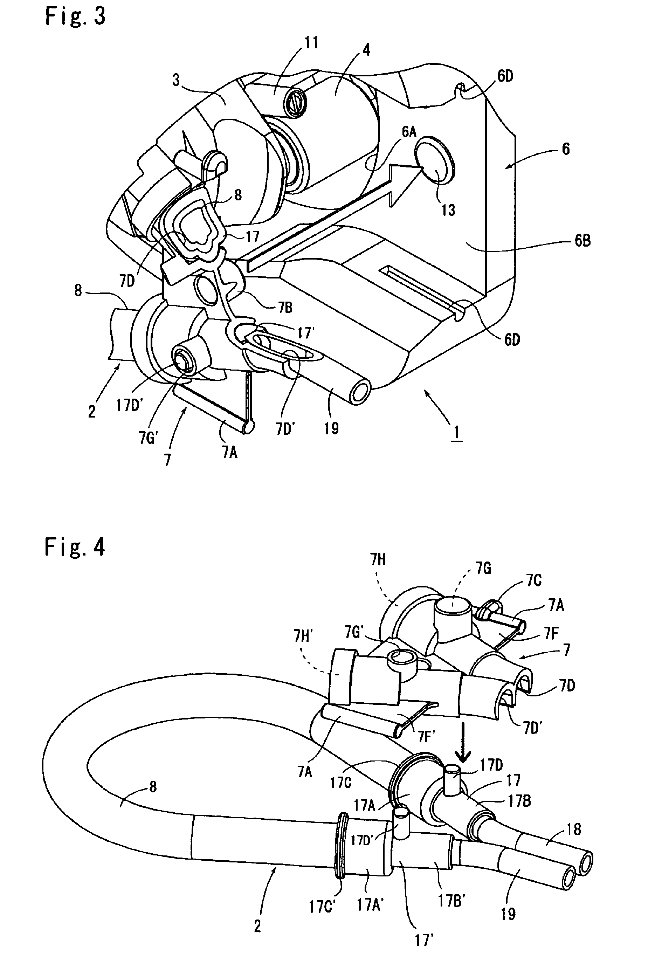

Furthermore, at a center of a bottom surface of the notch portion 6B, a detection switch 13 is arranged, and when the engagement portions 7A and 7A of the holder 7 are engaged with the engagement grooves 6D and 6D as described above, a projection portion 7B provided at a center of a back surface of the holder 7 comes into contact with the detection switch 13 (see Figure 3). At that time, a detection signal from the detection switch 13 is sent out the non-illustrated control device, enabling the control device to recognize the attachment of the duct 2 to the notch portion 6B of the housing 6 via the holder 7.

Furthermore, at the bottom surface of the notch portion 6B, a columnar reject pin 14, which can advance/retract toward the outside of the dialysis apparatus, is provided, and the reject pin 14 is made to advance/retract by means of a motor 15 provided in the main body portion of the dialysis apparatus.

When detaching the holder 7 holding the duct 2 from the notch portion 6B of the housing 6, the control device makes the reject pin 14 project toward the outside of the front surface of the dialysis apparatus by a predetermined amount from the bottom surface of the notch portion 6B via the motor 15. Consequently, the holder 7 and the duct 2 held by the holder 7 are forcibly pushed out toward the outside of the dialysis apparatus by means of a tip of the reject pin 14, whereby the engagement portions 7A and 7A of the holder 7 are separated from the engagement grooves 6D and 6D of the notch portion 6B. As described above, the holder 7 is detached from the notch portion 6B of the housing 6 via the reject pin 14.

An operation to detach the holder 7 from the notch portion 6B of the housing 6 by means of projection of the reject pin 14 may be performed by an operator operating a control panel of the dialysis apparatus, or may be incorporated in a final process in performing a series of dialysis treatment programs in the dialysis apparatus.

As illustrated in Figures 4 to 6, the duct 2 according to the present embodiment includes: the large-diameter tube 8, which is a looped curved portion to be attached to the cylindrical sidewall 6A of the housing 6; a supply tube 18 connected to an end of the large-diameter tube 8 via a supply-side connector 17 having a stepped cylindrical shape; and a discharge tube 19 connected to another end of the large-diameter tube 8 via a discharge-side connector 17' having a stepped tubular shape.

For the supply tube 18 and the discharge tube 19, those having a same diameter are used, and for the large-diameter tube 8, one having a diameter that is larger than that of the tubes 18 and 19. Since the large-diameter tube 8, which have a looped curved portion, is pressed and squeezed by the respective rollers 4 and 4, made of a soft material, such as polyvinyl chloride, in order to ensure the flow of a fluid (blood) and the endurance. Furthermore, both the supply tube 18 and the discharge tube 19 also made of polyvinyl chloride.

As illustrated in Figure 4, since the connectors 17 and 17' made of polyvinyl chloride in a same shape and dimensions, here, a configuration of the supply-side connector 17 will be described. In other words, the supply-side connector 17 includes a large-diameter connection portion 17A in which the end of the large-diameter tube 8 is fitted, a small-diameter connection portion 17B connected to the large-diameter connection portion 17A, in which an end of the supply tube 18 is fitted, a flange portion 17C, which is a ring-shaped projection formed at an outer peripheral portion, which is a free end, of the large-diameter connection portion 17A, and a columnar projection portion 17D projected from a position in an outer peripheral portion of the small-diameter connection portion 17B adjacent to the large-diameter connection portion 17.

The flange portion 17C is configured to engage with an engagement groove 7H on the later-described holder 7 side. Furthermore, the projection portion 17D is formed so as to project in a direction perpendicular to an axis of the supply-side connector 17, and the projection portion 17D is configured to engage with a cylindrical recess portion 7G on the holder 7 side.

Although the above description has been provided in terms of the configuration of the supply-side connector 17, since the discharge-side connector 17' is formed so as to have a same shape as that of the supply-side connector 17 as described above, a detailed description of the discharge-side connector 17' will be omitted. For the discharge-side connector 17', components corresponding to those of the supply-side connector 17 are provided with the same reference numerals as those of the supply-side connector 17 with an apostrophe added to each of the reference numerals.

Furthermore, the above-described configuration of the connectors 17 and 17' is one in the conventional art, and in the present embodiment the opposite ends of the large-diameter tube 8 are connected to the supply tube 18 and the discharge tube 19 via the existing connectors 17 and 17' as described above, and the connectors 17 and 17', which are connection portions, are attachably/detachably held by the holder 7. Furthermore, the projection portions 17D and 17D' of the connectors 17 and 17' may have, e.g., a polygonal columnar shape, a conical shape or a pyramid shape instead of the above-described columnar shape, and only need to have a shape that is engageable with the cylindrical recess portions 7G and 7G' on the holder 7 side.

Before the duct 2 according to the present embodiment being held by the holder 7, the tubes 8, 18 and 19 are connected as described below. In other words, an adhesive is applied to an outer peripheral portion of an end of the large-diameter tube 8 in advance and then fitting the end of the large-diameter tube 8 into the large-diameter connection portion 17A of the supply-side connector 17, and an adhesive is applied to an outer peripheral portion of an end of the supply tube 18 and then fitting the end of the supply tube 18 into the small-diameter connection portion 17B of the supply-side connector 17. Furthermore, an adhesive is applied to an outer peripheral portion of the other end of the large-diameter tube 8 and then fitting the other end of the large-diameter tube 8 into the large-diameter connection portion 17A' of the discharge-side connector 17', and an adhesive is applied to an outer peripheral portion of an end of the discharge tube 19 and then fitting the end of the discharge tube 19 into the small-diameter connection portion 17B' of the connector 17'. Consequently, the tubes 8, 18 and 19 are connected in series via the connectors 17 and 17', forming the duct 2. Also, in the present embodiment, when curving the large-diameter tube 8 to form a looped shape, thereby providing the curved portion, positions of the attached connectors 17 and 17' with reference to their respective axes are made to correspond to each other so that the projection portions 17D and 17D' of the connectors 17 and 17' are parallel to each other at positions adjacent to each other (see Figure 4).

Furthermore, the length of the large-diameter tube 8, which provides the curved portion, is set to have a dimension that is at little longer than a circumferential length of the cylindrical sidewall 6A of the housing 6. Since the large-diameter tube 8 is set to have such dimension, upon attachment of the holder 7 holding the connectors 17 and 17' to the notch portion 6B of the housing 6, the large-diameter tube 8, which is the curved portion, can be laid on the cylindrical sidewall 6A with some allowance.

Consequently, the tube pump 1 according to the present embodiment is **characterized in that** the connectors 17 and 17', which are portions connecting the large-diameter tube 8 and the tubes 18 and 19 of the duct 2, respectively, are held by the holder 7, and the held holder 7 can be attached to/from the notch portion 6B of the housing 6.

In other words, as illustrated in Figures 4 to 6, the holder 7 according to the present embodiment includes a single-piece synthetic resin, and is formed to have a size enabling outer peripheral surfaces of the connectors 17 and 17' to be covered on the outside of the front surface of the dialysis apparatus. The holder 7 includes: a pair of right and left stepped engagement recess portions 7D and 7D' capable of holding the connectors 17 and 17' and the tubes 18 and 19 adjacent to the respective connectors 17 and 17'; a central flat portion 7E connecting the stepped engagement recess portions 7D and 7D'; and a pair of right and left flat portions 7F and 7F' formed at side portions on the outer sides of the stepped engagement recess portions 7D and 7D'.

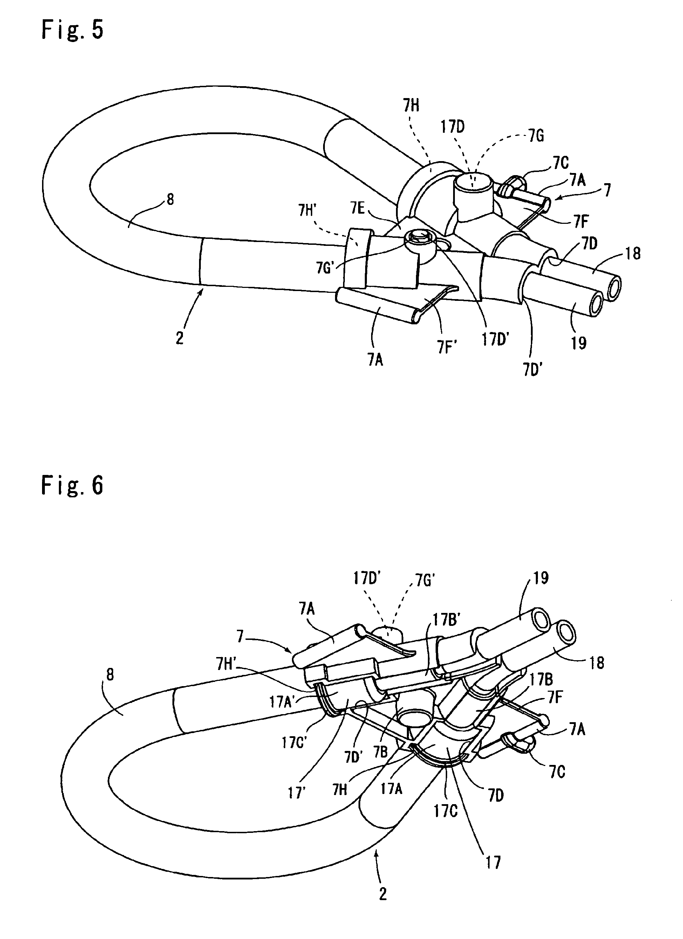

The stepped engagement recess portions 7D and 7D' are formed so to be symmetrical to each other and have same dimensions, and inner surface sides of the stepped engagement recess portions 7D and 7D' (the sides facing the connectors 17 and 17') each have an opening extending over the entire area in an axial direction thereof (see Figure 6). The connector 17 and 17' are held by fitting the outer peripheral portions of the connectors 17 and 17' and the outer peripheral portions of the tubes 18 and 19 located adjacent to the connectors 17 and 17' in the stepped engagement recess portions 7D and 7D' via the openings.

A stepped recess portion of each of the stepped engagement recess portions 7D and 7D' has a shape corresponding to shapes and dimensions of the peripheral surfaces of the flange portion 17C (17C'), the large-diameter connection portion 17A (17A') and the small-diameter connection portion 17B (17B') of the connector 17 or 17' and the tube 18 or 19. The axes of main portions of the stepped engagement recess portions 7D and 7D', which engage with the connectors 17 and 17', mutually form a predetermined angle, forming a V-shape, axes of auxiliary portions of the stepped engagement recess portions 7D and 7D', which engage with the tubes 18 and 19, are parallel to each other.

Accordingly, upon fitting the connectors 17 and 17' and the tubes 18 and 19 located adjacent thereto in the respective stepped engagement recess portions 7D and 7D', the connectors 17 and 17' are held by the holder 7 on a same plane, forming a V-shape, and the tubes 18 and 19 are held on a same plane in parallel to each other.

At ends on the large-diameter tube 8 side of the respective stepped engagement recess portions 7D and 7D', engagement grooves 7H and 7H' extending in a circumferential direction, which are engageable with respective flange portions 17C and 17C' of the connectors 17 and 17', are formed. Furthermore, at predetermined positions in the axial directions of the respective stepped engagement recess portions 7D and 7D', cylindrical recess portions 7G and 7G', which are engageable with the projection portions 17D and 17D' of the respective connectors 17 and 17', are provided so as to project toward the outside. Here, one cylindrical recess portion 7G includes a hole having a bottom while the other cylindrical recess portion 7G' has a cylindrical shape having no bottom. Consequently, an operator can visually confirm the direction of the holder 7 engaged with the projection portions 17D and 17D' of the connectors 17 and 17'. It is also possible that the cylindrical recess portion 7G' includes a hole having a bottom while the cylindrical recess portion 7G having a cylindrical shape having no bottom, and furthermore, it is possible that both of the cylindrical recess portions 7G and 7G' include a hole having a bottom, or have a cylindrical shape having no bottom.

As described above, upon fitting the connectors 17 and 17' and the tubes 18 and 19 located adjacent thereto in the respective stepped engagement recess portions 7D and 7D', the flange portions 17C and 17C' of the connectors 17 and 17' engage with the respective engagement grooves 7H and 7H', while the projection portions 17D and 17D' of the connectors 17 and 17' engaging with the cylindrical recess portions 7G and 7G' (see Figures 4-6).

As described above, the holder 7 according to the present embodiment is configured so that one stepped engagement recess portion 7D is fitted on the supply-side connector 17 and the supply tube 18 located adjacent thereto to hold the supply-side connector 17, while the supply tube 18, while the other stepped engagement recess portion 7D' being fitted on the discharge-side connector 17' and the discharge tube 19 located adjacent thereto to hold the discharge-side connector 17'. Here, one engagement groove 7H of the holder 7 engages with the flange portion 17C of the supply-side connector 17, while the other engagement groove 7H' of the holder 7 engaging with the flange portion 17C' of the discharge-side connector 17'. Furthermore, one cylindrical recess portion 7G of the holder 7 engages with the projection portion 17D of the supply-side connector 17, while the other cylindrical recess portion 7G' of the holder 7 engaging with the projection portion 17D' of the discharge-side connector 17' (see Figures 4 to 6). Consequently, the positions in the longitudinal direction of attachment of the tubes 18 and 19 are determined, and the connectors 17 and 17' are held on a same plane, forming a V-shape (see Figures 4 and 5).

Furthermore, the central flat portion 7E and the flat portions 7F and 7F' on the outer sides of the holder 7 are formed so as to be on a same plane, and edge portions of the flat portions 7F and 7F' are kept in parallel to each other. The edge portions of the flat portions 7F and 7F' are respectively formed to have a columnar shape, forming linear engagement portions 7A and 7A. As described above, the holder 7 can be attached to the housing 6 by engaging the engagement portions 7A and 7A with the engagement grooves 6D and 6D in the housing 6.

Furthermore, in order to prevent upside down insertion of the duct the positioning projection 7C is formed at a predetermined position in the longitudinal direction of the engagement portion 7A on the supply tube 18 side. The position in the horizontal direction of the holder 7 engaged with the housing 6 can be restricted by engaging the positioning projection 7C of the holder 7 with the positioning recess portion 6E on the housing 6 side.

Furthermore, on the inner surface side of the central flat portion 7E of the holder 7, a substantially columnar projection portion 7B is provided so as to project inward. Upon engaging the holder 7 holding the connectors 17 and 17' with the notch portion 6B of the housing 6 to completely engage the engagement portions 7A and 7A with the engagement grooves 6D and 6D, the projection portion 7B of the holder 7 comes into contact with the detection switch 13 on the housing 6 side (see Figure 3). Consequently, the aforementioned control device in the dialysis apparatus can recognize the attachment of the duct 2 to the housing 6.

A process of operation where the tube pump 1 according to the present embodiment, which includes the above-described configuration, is used as a blood pump for a dialysis apparatus will be described.

In this case, first, in a preparatory process before use, the large-diameter tube 8, the supply tube 18 and the discharge tube 19 included in the duct 2 are connected via the connectors 17 and 17'.

Furthermore, the large-diameter tube 8 of the duct 2 is curved to form a looped curved portion and the connectors 17 and 17' are positioned adjacent to the large-diameter tube 8, and in such a state, the stepped engagement recess portions 7D and 7D' of the holder 7 are engaged with outer surfaces of the connectors 17 and 17' and outer faces of the tubes 18 and 19 on the side where the projection portions 17D and 17D' of the connectors 17 and 17' are provided (see Figures 4 and 5). Furthermore, the engagement grooves 7H and 7H' of the stepped engagement recess portions 7D and 7D' are engaged with the flange portions 17C and 17C' of the connectors 17 and 17', while engaging the cylindrical recess portions 7G and 7G' of the stepped engagement recess portions 7D and 7D' with the projection portions 17D and 17D' of the connectors 17 and 17'. Consequently, the supply-side connector 17 and the supply tube 18 are held in the stepped engagement recess portion 7D of the holder 7, while the discharge-side connector 17' and the discharge tube 19 being held in the other stepped engagement recess portion 7D'.

As described above, in the preparatory process, for loading the duct 2 with the connectors 17 and 17' held, the access cover 12 of the housing 6 is opened and the holder 7 in the aforementioned state is engaged with the notch portion 6B of the housing 6. Here, first, the positioning projection 7C of the holder 7 is engaged with the positioning recess portion 6E, the entire holder 7 is then pushed into the notch portion 6B, and then, the engagement portions 7A and 7A are engaged with the engagement grooves 6D and 6D.

Consequently, attachment of the holder 7 holding the connectors 17 and 17' of the duct 2 to the notch portion 6B of the housing 6 is completed. Upon the completion of the attachment as described above, the projection portion 7B of the holder 7 is brought into contact with the detection switch 13, enabling the non-illustrated control device to recognize the attachment of the duct 2 to the housing 6.

Furthermore, in a state in which the connectors 17 and 17' of the duct 2 are attached to the notch portion 6B of the housing 6 by means of the holder 7, the looped large-diameter tube 8 does not engage into the cylindrical sidewall 6A and is laid on the outer peripheral portion of the rotor 3 on the outside of the front surface of the dialysis apparatus. Thus, the guide pins 11 of the rotor 3 are positioned exterior to parts of the large-diameter tube 8 adjacent to the holder 7 in the dialysis apparatus.

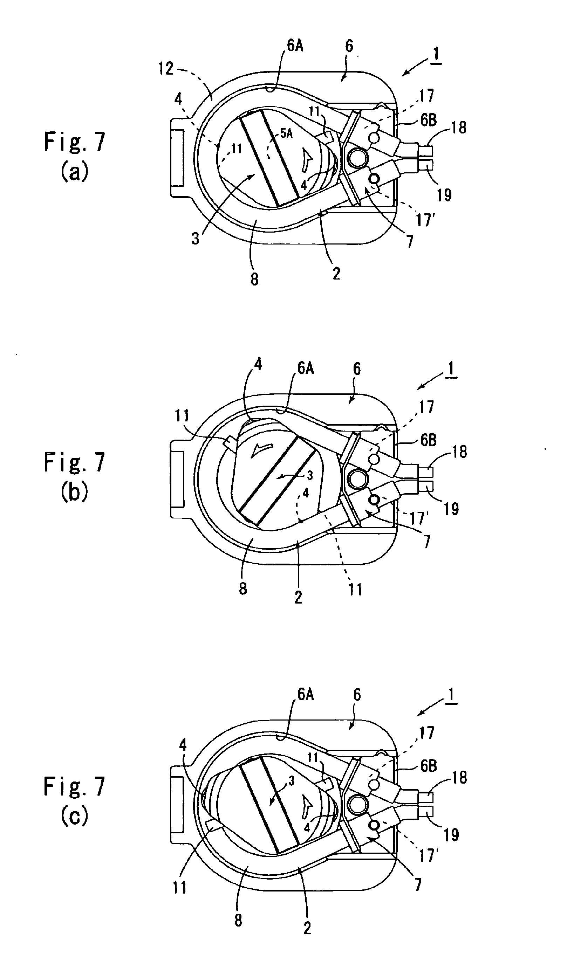

Subsequently, an operator operates the control panel of the dialysis apparatus to turn the rotor 3 counterclockwise in Figure 1 via the motor 5, resulting in the guide pins 11 of the rotor 3, which is turned, fitting the substantially entire region of the looped large-diameter tube 8 between the cylindrical sidewall 6A and the outer peripheral portion of the rotor 3 and the rollers 4 (see Figures 7(a) to 7(c)). In the present embodiment, as described above, the large-diameter tube 8 of the duct 2 can automatically be attached to the cylindrical sidewall 6A of the housing 6 via the rotor 3 and the guide pins 11 and 11 provided with the rotor 3. Upon the attachment as described above, the large-diameter tube 8 is sandwiched between the rollers 4 and 4 of the rotor 3 and parts of the cylindrical sidewall 6A at positions facing the rollers 4 and 4. After the completion of attachment of the large-diameter tube 8, the connectors 17 and 17' and the tubes 18 and 19 of the duct 2 to the housing 6 via the holder 7 as described above, the operator closes the access cover 12. Subsequently, the operator connects non-illustrated other ends of the supply tube 18 and the discharge tube 19 to relevant parts.

Subsequently, the dialysis apparatus operates according to a predetermined operation program to perform a dialysis treatment. Accordingly, the rotor 3 is rotated via the motor 5, and the large-diameter tube 8 is thereby squeezed by the rollers 4 and 4, allowing blood to be sent via the duct 2.

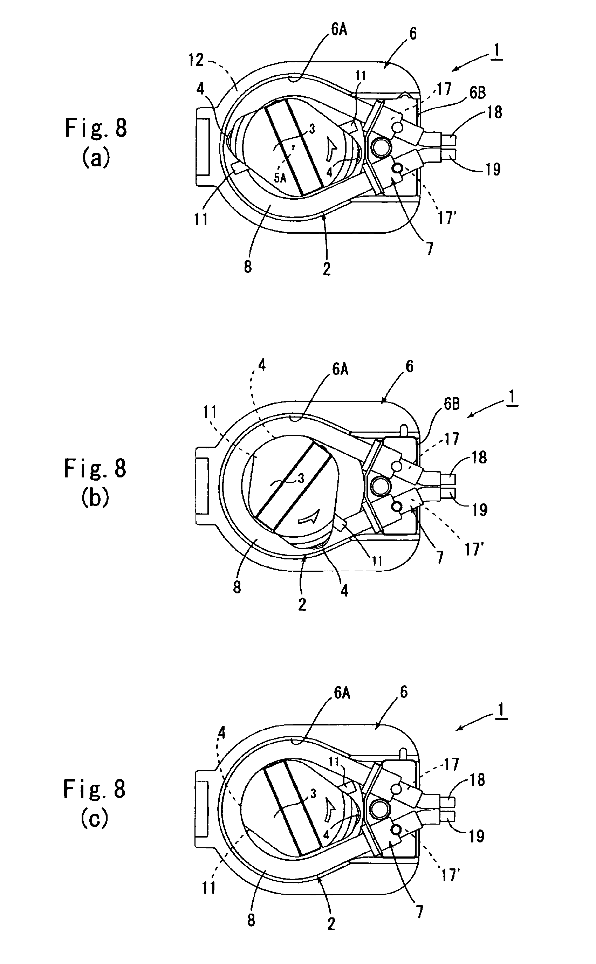

After the dialysis operation, detachment of the holder 7 is performed as described below. First, the control device makes the reject pin 14 project via the motor 15. Thus, the holder 7 is pushed toward the outside of the dialysis apparatus by a predetermined distance by means of the reject pin 14, resulting the engagement portions 7A and 7A of the holder 7 being separated from the engagement grooves 6D and 6D of the housing 6. Then, the connectors 17 and 17' float exterior to the guide pins 11 and 11 of the rotor 3 in the dialysis apparatus, and in such a state, the rotor 3 is rotated via the drive shaft 5A of the motor 5. Consequently, the guide pins 11 burrow into between the large-diameter tube 8 and the cylindrical bottom surface of the housing 6 while being rotated by the rotor 3, allowing the entire large-diameter tube 8 to be pushed from between the cylindrical sidewall 6A and the rollers 4 and 4 toward the outside of the dialysis apparatus (see Figures 8(a) to 8(c)). As described above, in the present embodiment, the large-diameter tube 8, which is a curved portion, can automatically attached/detached to/from the cylindrical sidewall 6A by means of the rotor 3 and the guide pins 11.

The tube pump 1 according to the present embodiment described above includes the holder 7 attachably/detachably holding the blood supply-side and blood discharge-side connectors 17 and 17' of the duct 2. The connectors 17 and 17' are held by the holder 7, and the holder 7 in such a state is attached/detached to/from the housing 6.

Thus, the duct 2 can precisely be attached to the notch portion 6B, which is a predetermined attachment position, of the housing 6. Furthermore, the supply-side and discharge-side parts (the connectors 17 and 17') of the duct 2 are attached to the housing 6 via the holder 7, enabling easy attachment/detachment of the duct 2 to/from the housing 6.

Furthermore, in the present embodiment, the flange portions 17C and 17C' of the connectors 17 and 17' engage with the engagement grooves 7H and 7H' of the holder 7, while the projection portions 17D and 17D' engaging with the cylindrical recess portions 7G and 7G'. Thus, even if a longitudinal tensile force is imposed on the duct 2 when the rotor 3 is rotated and the large-diameter tube 8 is thereby squeezed by the rollers 4, it is possible to favorably prevent the duct 2 from being moved in the longitudinal direction. Furthermore, the engagement of the projection portions 17D and 17D' with the cylindrical recess portions 7G and 7G' enables the duct 2 itself to be prevented from being rotated with reference to the axis.

Furthermore, the holder 7 according to the present embodiment holds the connectors 17 and 17', that is, the parts of connection between the opposite ends of the large-diameter tube 8 and the tubes 18 and 19 on the outer sides, eliminating the need to form a fluid passage in the holder 7 itself. Thus, comparing with the holders including a fluid passage inside, which have been described above, the configuration of the holder 7 can be simplified. Thus, a mold for fabricating the holder 7 can be simplified compared to the conventional cases, and accordingly, the manufacturing costs of the tube pump 1 can be reduced by that amount. Furthermore, since no fluid passage allowing a fluid to flow therein is provided in the holder 7 itself, maintenance of the holder 7 is very easy compared to the conventional product disclosed in Patent Literature 1.

Furthermore, in the present embodiment, existing connectors each including a projection portion 17D (17D') are used as the connectors 17 and 17', and thus, the holder 7 holding the connectors 17 and 17' can repeatedly be used in order to hold the holders 17 and 17' of the duct 2. In that sense, also, the tube pump 1 according to the present embodiment enables the manufacturing costs to be reduced compared to the conventional art.

Although in the above-described embodiment, the flange portions 17C and 17C', which are first engagement portions, of the connectors 17 and 17', are engaged with the engagement grooves 7H and 7H', which are second engagement portions, of the holder 7, while the projection portions 17D and 17D', which are also first engagement portions, being engaged with the cylindrical recess portions 7G and 7G', which are also second engagement portion, of the holder 7, a configuration such as described below may be employed. In other words, the flange portions 17C and 17C', which are first engagement portions, of the connectors 17 and 17' in the above-described embodiment may be omitted while the engagement grooves 7H and 7H', which are second engagement portions, of the holder 7 being omitted. Alternatively, contrary to such configuration, the projection portions 17D and 17D', which are first engagement portions, of the connectors 17 and 17' in the above-described embodiment may be omitted while the cylindrical recess portions 7G and 7G', which are second engagement portions, of the holder 7 being omitted. In other words, a configuration in which at least a first engagement portion is formed in each of the connectors 17 and 17' and second engagement portions that are engageable with the respective first engagement portions of the holder 7 are formed may be employed. Furthermore, the recess portion-projection portion relationship for the first engagement portions and the second engagement portions engaging with the first engagement portions may be opposite from that of the above-described embodiment. For example, recess portions, which are first engagement portions, may be formed on the duct 2 while projection portions, which are second engagement portions, being formed on the holder 7.

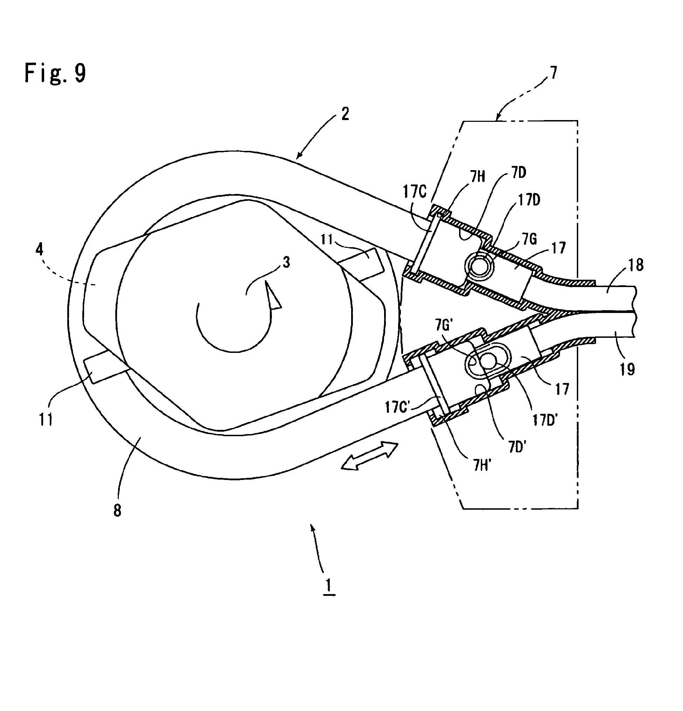

Next, Figure 9 is a configuration diagram illustrating a major portion of a tube pump 1 according to a second embodiment of the present invention. In the second embodiment, the stepped engagement recess portion 7D' on the discharge side of the holder 7 is improved to tolerate variations in the length of the large-diameter tube 8. More specifically, a columnar recess portion 7G', which extends in a longitudinal direction of a duct 2 (discharge tube 19) and has the shape of an elongated hole, is formed at a stepped engagement recess portion 7D' on the discharge tube 19 side of a holder 7 instead of the above-described cylindrical recess portion. Furthermore, a length in an axial direction of an engagement groove 7H' of the stepped engagement recess portion 7D', which engages with a flange portion 17C' of a discharge-side connector 17' is set to be several times a length in an axial direction of the flange portion 17C'. The rest of configuration is no different from the configuration of the tube pump 1 according to the above-described first embodiment.

According to such second embodiment, an allowance is provided to the length in the axial direction of each of the columnar recess portion 7G' and the engagement groove 7H' so as to, when an outer surface of the discharge-side connector 17' is engaged with the stepped engagement recess portion 7D' of the holder 7, select the position in the axial direction of the engaged discharge-side connector 17' within a predetermined range. Thus, according to the second embodiment, the connectors 17 and 17' can be held by the holder 7 in such a manner that variations in the length of the large-diameter tube 8, which is a curved portion, of the duct 2 are tolerated, enabling the holder 7 in such a state to be attached to a notch portion 6B of a housing 6.

The second embodiment enables provision of operation and effects similar to those of the above-described first embodiment, and furthermore, suppression of manufacturing costs of the duct 2 because the large-diameter tube 8 can be attached to the housing 6 via the holder 7 as long as the entire length of the large-diameter tube 8 falls within the predetermined range.

Although in the above-described embodiments, the engagement portions 7A and 7A are provided in parallel to each other at end portions of the holder 7, and accordingly, the engagement grooves 6D and 6D of the housing 6 are provided also in parallel to each other, a configuration such as described below can be employed as another embodiment. In other words, it is possible to form engagement portions 7A and 7A of a holder 7 so as to form a predetermined angle, and accordingly, form engagement grooves 6D and 6D of a housing 6 so as to also form the predetermined angle. For example, where the engagement portions 7A and 7A are formed so as to form a shape like a separated "V" so that the angle formed by the engagement portions 7A and 7A decreases toward a rotor 3 in the housing 6 and the engagement grooves 6D and 6D are also formed so as to form a shape like a separated "V" in accordance with the engagement portions 7A and 7A, an effect such as described below can be provided. When the duct 2 is pulled toward the rotor 3 as a result of rotation of the rotor 3, the engagement portions 7A and 7A and the engagement grooves 6D and 6D, which each form a shape like a separated "v", functions as a stopper, enabling reliable prevention of the holder 7 and a supply tube 18 of the duct 2 from being pulled toward the rotor 3.

Furthermore, although the above description has been provided in terms of a case where the tube pump 1 according to each of the present embodiments is used as a blood pump for a dialysis apparatus, it should be understood that the tube pump 1 according to each of the present embodiments can also be used as a pump for pumping, e.g., saline.

### Reference Signs List

- 1: tube pump
- 2: duct
- 3: rotor
- 4, 4: roller
- 6: housing
- 6A: cylindrical sidewall
- 7: holder
- 8: large-diameter tube (curved portion)
- 18: supply tube
- 19: discharge tube
- 17C, 17C': flange portion (first engagement portion)
- 7H, 7H': engagement groove (second engagement portion)
- 17D, 17D': projection portion (first engagement portion)
- 7G, 7G': cylindrical recess portion (second engagement portion)

## Claims

1. A tube pump (1) comprising:
a housing (6) including a cylindrical side wall (6A) in an inner portion thereof;
a rotor (3) including a roller (4) arranged on an inward side of the cylindrical side wall (6A) of the housing (6), the rotor (3) being rotated by a drive source (5);
a duct (2) including a curved portion (8) for attachment between the cylindrical side wall (6A) and the roller (4), the tube pump (1) being configured to rotate the rotor (3) to squeeze the curved portion (8) via the roller (4) thereby pumping a fluid via the duct (2), wherein the duct is provided with a supply tube (18) connected to an end of the curved portion (8) via a supply-side connector (17) and a discharge tube (19) connected to the other end of the curved portion (8) via a discharge-side connector (17'); and
a single holder (7) for engagement with each of said connectors (17, 17'),
**characterised in that**:
the holder (7) has a recess portion (7D, 7D') for engagement with each of said connectors (17, 17'), the holder (7) having a pair of right and left flat portions (7F, 7F') formed on an outer side of the recess portion (7D, 7D'), each flat portion extending to a respective edge portion having a linear engagement portion (7A);
the housing (6) has a notch portion (6B) having a pair of opposing side walls extending from the cylindrical side walls (6A), each sidewall having a respective engagement groove (6D) extending in a direction intersecting the axis of the drive shaft (5A), each engagement groove (6D) for receiving a respective linear engagement portion (7A) for releaseably securing the holder (7) to the housing (6) when the connectors (17,17') are held in the recess portion (7D, 7D') of the holder.

2. The tube pump according to claim 1, wherein the notch portion (6B) of the housing includes a detection switch (13) for detecting that the holder (7) is mounted on the notch portion and a reject pin (14) for applying a force to the holder to eject it from the housing.

## Patentansprüche

1. Rohrpumpe (1), umfassend:
ein Gehäuse (6) mit einer zylindrischen Seitenwand (6A) in einem Innenabschnitt davon;
einen Rotor (3) mit einer Walze (4), die auf einer Innenseite der zylindrischen Seitenwand (6A) des Gehäuses (6) angeordnet ist, wobei der Rotor (3) durch eine Antriebsquelle (5) gedreht wird;
eine Leitung (2) mit einem gekrümmten Abschnitt (8) zur Anbringung zwischen der zylindrischen Seitenwand (6A) und der Walze (4), wobei die Rohrpumpe (1) konfiguriert ist, den Rotor (3) zu drehen, um den gekrümmten Abschnitt (8) über die Walze zusammenzuquetschen, wodurch ein Fluid durch die Leitung gepumpt wird, wobei die Leitung mit einem Versorgungsrohr (18), das über ein Versorgungsseiten- Verbindungselement (17) mit einem Ende des gekrümmten Abschnitts (8) verbunden ist, und einem Abgaberohr (19) bereitgestellt ist, das über ein Abgabenseiten-Verbindungselement (17') mit dem anderen Ende des gekrümmten Abschnitts (8) verbunden ist; und
eine einzelne Halterung (7) zum Ineingriffbringen mit jedem der Verbindungselemente (17, 17'),
**dadurch gekennzeichnet, dass**:
die Halterung (7) einen Aussparungsabschnitt (7D, 7D') zum Ineingriffbringen mit jedem der Verbindungselemente (17, 17') umfasst, wobei die Halterung (7) ein Paar aus einem rechten und einem linken flachen Abschnitt (7F, 7F') aufweist, wobei jeder flache Abschnitt sich bis zu einem entsprechenden Kantenabschnitt mit einem linearen Eingriffsabschnitt (7A) erstreckt; und dass
das Gehäuse (6) einen Vertiefungsabschnitt (6B) mit einem Paar von gegenüberliegenden Seitenwänden, die sich von den zylindrischen Seitenwänden (6A) erstrecken, aufweist, wobei jede Seitenwand eine entsprechende Eingriffsnut (6D) aufweist, die sich in einer Richtung erstreckt, die die Achse der Antriebswelle (5A) kreuzt, wobei jede Eingriffsnut (6D) dem Empfangen eines entsprechenden linearen Eingriffsabschnitts (7A) zum lösbaren Festlegen der Halterung (7) auf dem Gehäuse (6) dient, wenn die Verbindungselemente (17, 17') im Aussparungsabschnitt (7D, 8D') der Halterung gehalten sind.

2. Rohrpumpe nach Anspruch 1, wobei der Vertiefungsabschnitt (6B) des Gehäuses einen Detektionsschalter (13) zum Detektieren, ob die Halterung (7) auf dem Vertiefungsabschnitt befestigt ist, und einen Ausstoßstift (14) zum Beaufschlagen einer Kraft auf die Halterung umfasst, um diese aus dem Gehäuse auszustoßen.

## Revendications

1. Pompe à tube (1) comprenant :
un boîtier (6) comprenant une paroi latérale cylindrique (6A) dans sa partie interne ;
un rotor (3) comprenant un rouleau (4) agencé sur un côté interne de la paroi latérale cylindrique (6A) du boîtier (6), le rotor (3) étant entraîné en rotation par une source d'entraînement (5) ;
un conduit (2) comprenant une partie incurvée (8) pour la fixation entre la paroi latérale cylindrique (6A) et le rouleau (4), la pompe à tube (1) étant configurée pour faire tourner le rotor (3) afin de comprimer la partie incurvée (8) via le rouleau (4), pompant ainsi un fluide via le conduit (2), dans laquelle le conduit est prévu avec un tube d'alimentation (18) raccordé à une extrémité de la partie incurvée (8) via un connecteur (17) du côté de l'alimentation et un tube de refoulement (19) raccordé à l'autre extrémité de la partie incurvée (8) via un connecteur (17') du côté du refoulement ; et
un seul support (7) pour la mise en prise avec chacun desdits connecteurs (17, 17'),
**caractérisée en ce que** :
le support (7) a une partie d'évidement (7D, 7D') pour la mise en prise avec chacun desdits connecteurs (17, 17'), le support (7) ayant une paire de parties plates droite et gauche (7F, 7F') formées sur un côté externe de la partie d'évidement (7D, 7D'), chaque partie plate s'étendant vers une partie de bord respective ayant une partie de mise en prise linéaire (7A) ;
le boîtier (6) a une partie d'encoche (6B) ayant une paire de parois latérales opposées s'étendant à partir des parois latérales cylindriques (6A), chaque paroi latérale ayant une rainure de mise en prise (6D) respective s'étendant dans une direction coupant l'axe de l'arbre d'entraînement (5A), chaque rainure de mise en prise (6D) étant prévue pour recevoir une partie de mise en prise linéaire (7A) respective pour fixer de manière amovible le support (7) sur le boîtier (6) lorsque les connecteurs (17, 17') sont maintenus dans la partie d'évidement (7D, 7D') du support.

2. Pompe à tube selon la revendication 1, dans lequel la partie d'encoche (6B) du boîtier comprend un commutateur de détection (13) pour détecter que le support (7) est monté sur la partie d'encoche et une broche de rejet (14) pour appliquer une force sur le support afin de l'éjecter du boîtier.
